# EUROPÄISCHE PATENTANMELDUNG

(11) **EP 0 148 498 A1**
(43) Veröffentlichungstag der Anmeldung: **17.07.1985**
(21) Anmeldenummer: 84116306.6
(22) Anmeldetag: 27.12.1984
(51) Int. Cl.: C07D 239/42, C07D 239/46, C07D 251/46, A01N 47/44

(54) **Neue Sulfonylguanidin-Derivate**

(30) Priorität: 10.01.1984 JP 1257/84
(71) Anmelder: NIHON TOKUSHU NOYAKU SEIZO K.K., Chuo-ku Tokyo 103 (JP)
(72) Erfinder: Shiokawa, Kozo, Kawasaki-shi Kanagawa-ken (JP); Moriya, Koichi, Hachioji-shi Tokio (JP); Goto, Toshio, Sagamihara-shi Kanagawa-ken (JP); Kamochi, Atsumi, Hino-shi Tokyo (JP); Kohama, Shigeo, Hachioji-shi Tokyo (JP)
(74) Vertreter: Schumacher, Günter, Dr.

(57) **Zusammenfassung**

Die Erfindung betrifft neue Sulfonylguanidin-Derivate der allgemeinen Formel (I) 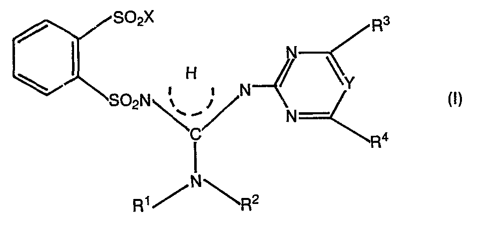 in der
X eine niedere Alkoxy-Gruppe, eine niedere Alkylamino-Gruppe oder eine Morpholino-Gruppe bezeichnet,
R¹ ein Wasserstoff-Atom oder die Gruppe 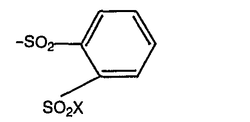 bezeichnet, in der X die oben angegebene Bedeutung hat, R² eine Hydroxy-Gruppe, eine niedere Alkoxy-Gruppe, eine Di-Niederalkylamino-Gruppe bezeichnet,
R³ und R⁴ jeweils für eine niedere Alkyl-Gruppe oder eine niedere Alkoxy-Gruppe stehen und
Y N oder CH bezeichnet,
Verfahren zu ihrer Herstellung und ihre Verwendung als Herbizide.

## Beschreibung

Die vorliegende Erfindung betrifft neue Sulfonylguanidin-Derivate, Verfahren zu ihrer Herstellung sowie ihre Verwendung als Herbizide.

Insbesondere betrifft die vorliegende Erfindung Sulfonylguanidin-Derivate der nachstehenden Formel (I) 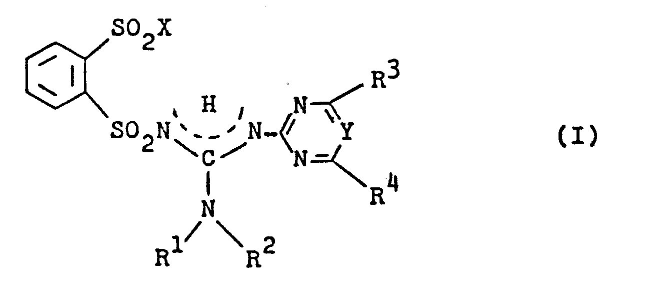 in der
X eine niedere Alkoxy-Gruppe, eine niedere Alkylamino-Gruppe oder eine Morpholino-Gruppe bezeichnet,
R¹ ein Wasserstoff-Atom oder die Gruppe 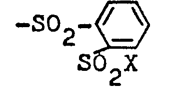 bezeichnet, in der X die oben angegebene Bedeutung hat,
R² eine Hydroxy-Gruppe, eine niedere Alkoxy-Gruppe, eine Di-Niederalkylamino-Gruppe bezeichnet,
_{R}³ und _{R}⁴ jeweils für eine niedere Alkyl-Gruppe oder
eine niedere Alkoxy-Gruppe stehen und Y N oder CH bezeichnet.

Die Verbindungen der allgemeinen Formel gemäß der vorliegenden Erfindung können beispielsweise mittels der folgenden Verfahren hergestellt werden, auf die sich die Erfindung ebenfalls ertreckt.

### Verfahren i)

Ein Verfahren zur Herstellung der Sulfonylguanidine der allgemeinen Formel (I) umfaßt die Umsetzung einer Verbindung der Formel 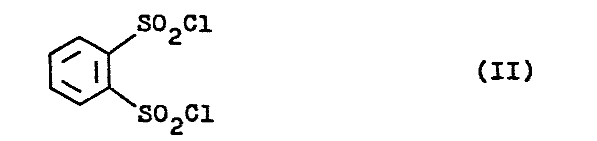 mit einer Verbindung der allgemeinen Formel 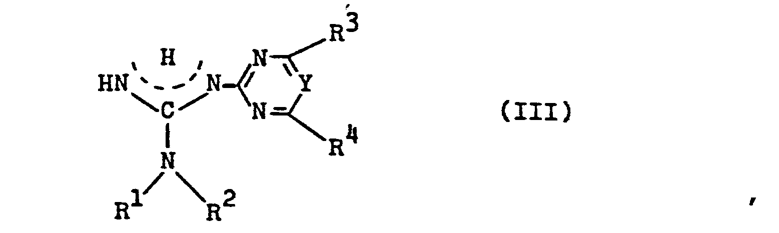 in der _{R}¹, _{R}², _{R}³, _{R}⁴ und Y die im Vorstehenden angegebenen Bedeutungen haben, und anschließend mit einer Verbindung der allgemeinen Formel 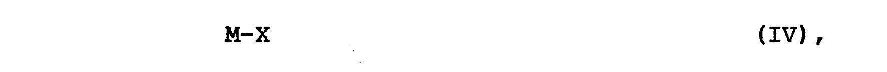 in der X die im Vorstehenden angegebene Bedeutung hat und M ein Wasserstoff-Atom oder ein Alkalimetall-Atom bezeichnet.

### Verfahren ii)

Ein Verfahren zur Herstellung der Sulfonylguanidin-Derivate der allgemeinen Formel (I) umfaßt die Umsetzung einer Verbindung der allgemeinen Formel 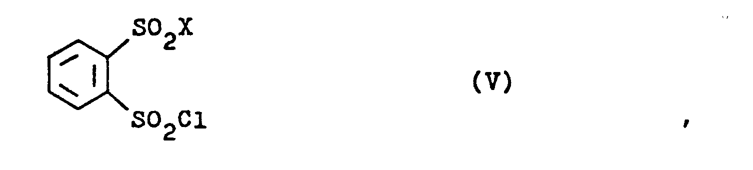 in der X die im Vorstehenden angegebene Bedeutung hat, mit einer Verbindung der oben angegebenen allgemeinen Formel (III) in Gegenwart einer Base.

### Verfahren iii)

Ein Verfahren zur Herstellung der Sulfonylguanidinₑ der allgemeinen Formel (I) umfaßt die Umsetzung einer Verbindung der allgemeinen Formel 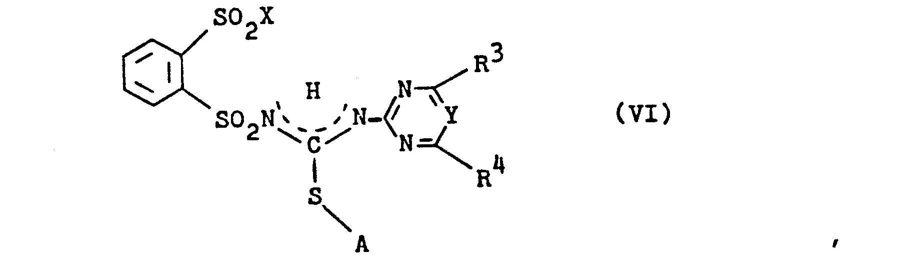 in der _{X}, _{R}³, _{R}⁴, _{Y} und M die im Vorstehenden angegebenen Bedeutungen haben und A eine niedere Alkyl-Gruppe bezeichnet, mit einer Verbindung der allgemeinen Formel 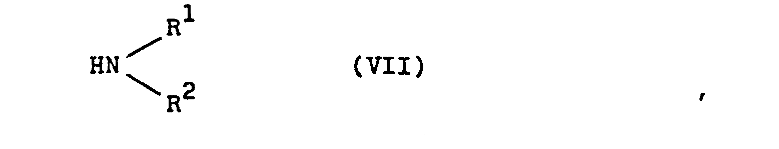 in der _{R}¹ und _{R}² die im Vorstehenden angegebenen Bedeutungen haben.

Die vorliegende Erfindung erstreckt sich auch auf Herbizide, die die Sulfonylguanidin-Derivate der allgemeinen Formel (I) als Wirkstoffe enthalten.

Seitens der Anmelderin wurden ausgedehnte Untersuchungen mit dem Ziel der Schaffung neuer Verbindungen mit herbizider Aktivität durchgeführt, und als ein Ergebnis dieser Arbeiten wurden die Sulfonylguanidin-Derivate der allgemeinen Formel (I) mit Erfolg synthetisiert. Weiterhin wurde gefunden, daß diese Verbindungen eine hervorragende herbizide Wirksamkeit aufweisen. Die vorliegende Erfindung beruht auf diesem Befund.

Nach bestem Wissen der Anmelderin sind die Sulfonylguanidin-Derivate der allgemeinen Formel (I) gemäß der vorliegenden Erfindung neue Verbindungen, die in vor dem Einreichungszeitpunkt der vorliegenden Anmeldung bekannten Veröffentlichungen nicht beschrieben sind. Die Verbindungen gemäß der vorliegenden Erfindung sind durch die Tatsache gekennzeichnet, daß sie biologisch eine hervorragende selektive herbizide Wirksamkeit insbesondere gegen Ackerunkräuter auf höher gelegenen Anbauflächen besitzen, ohne daß sie gegen die betreffenden Nutzpflanzen wie Sojabohnen und Winterweizen Phytotoxizität aufweisen, und daß die erwähnte herbizide Aktivität erstmals vermittels ihrer durch die allgemeine Formel (I) dargestellten chemischen Struktur erzielt werden kann.

Die Verbindungen gemäß der vorliegenden Erfindung können in einfacher Weise mittels der oben beschriebenen Verfahren i), ii) und iii) hergestellt werden.

Aus diesem Grunde ist es ein Ziel der vorliegenden Erfindung, neue Sulfonylguanidin-Derivate der allgemeinen Formel (I), Verfahren zur Herstellung derselben sowie ihre Verwendung als Herbizide verfügbar zu machen.

Dieses Ziel sowie weitere Ziele und Vorteile der vorliegenden Erfindung sind der folgenden Beschreibung zu entnehmen.

Die Verbindungen der vorliegenden Erfindung können mit Vorteil als Unkrautbekämpfungsmittel eingesetzt werden, weil sie nur geringe Toxizität gegenüber warmblütigen Tieren besitzen und keinerlei Phytotoxizität gegen Kulturpflanzen aufweisen, wenn sie in den üblichen Dosierungen angewandt werden, und somit eine gute Selektivität zeigen. Die Herbizide gemäß der vorliegenden Erfindung zeigen eine hervorragende selektive Bekämpfungswirksamkeit insbesondere beim Einsatz gegen ein breites Spektrum von Ackerunkräutern als Mittel zur Bodenbehandlung vor dem Auflaufen oder als Mittel zur Laub-/ Stengel- und Bodenbehandlung auf höher gelegenen Anbauflächen.

Wie oben dargelegt besitzen die Verbindungen gemäß der vorliegenden Erfindung hohe Sicherheit und zeigen eine herausragende herbizide Wirksamkeit.

Sie besitzen ein herbizides Spektrum gegen Alopecurus aequalis Sobol. var. amurensis Ohwi, Setaria glauca P.Beauv.,
Stellaria media Villars,
Echinochloa crus-galli P.Beauv.,
Digitaria adscendens Henr.,
Eleusine indica Gaertn.,
Digitaria violascens Link,
Amaranthus lividus Loisel.,
Polygonum blumei Meisn.,
Chenopodium album L.,
Chenopodium ficifolium Smith,
Amaranthus retroflexus L.,
Poa annua L.,
Rorippa palustris Bess.,
Polygonum nepalense Meisn.,
Rumex obtusifolius L.,
Rumex japonicus Houtt.,
Lamium amplexicaule L.,
Galium spurium L.,
Stellaria alsine Grimn.,
Cardamine flexuosa With. und
Polygonum ariculare L..

Die Verbindungen gemäß der vorliegenden Erfindung zeigen eine starke herbizide Wirkung auch gegenüber anderen als den oben beispielhaft genannten Unkräutern. Beispielsweise wurde beobachtet, daß sie ausgezeichnete herbizide Wirksamkeit und eine Hemmwirkung auf das Weiterwachsen schwierig zu bekämpfender perennierender Unkräuter, beispielsweise Cyperus rotundus L. und Cynodon dactylon Pers., ausüben.

Die Verbindungen gemäß der vorliegenden Erfindung können sicher und ohne Verursachung von Toxizität bei vielen Nutzpflanzen, darunter Bohnen, Weizen, Baumwolle, Möhren, Kartoffeln, Rüben, Kohl, Senf, Erdnüssen, Rettich, Tabak, Tomaten und Gurken angewandt werden.

Die Einsatzmöglichkeiten der aktiven Verbindungen gemäß der vorliegenden Erfindung sind nicht auf Ackerunkräuter auf höher gelegenen Anbauflächen beschränkt, sondern die Verbindungen sind ebenso auch gegen Schadunkräuter bei Reis, bei Mattenbinsen und auf Brachflächen wirksam. Der Begriff "Unkräuter", wie er hierin verwendet wird, bezeichnet im weitesten Sinne alle Pflanzen, die an Orten wachsen, an denen sie unerwünscht sind.

Die Verbindungen der allgemeinen Formel (I) gemäß der vorliegenden Erfindung können beispielsweise mittels der folgenden Verfahren i), ii) und iii) hergestellt werden.

### Verfahren (i)

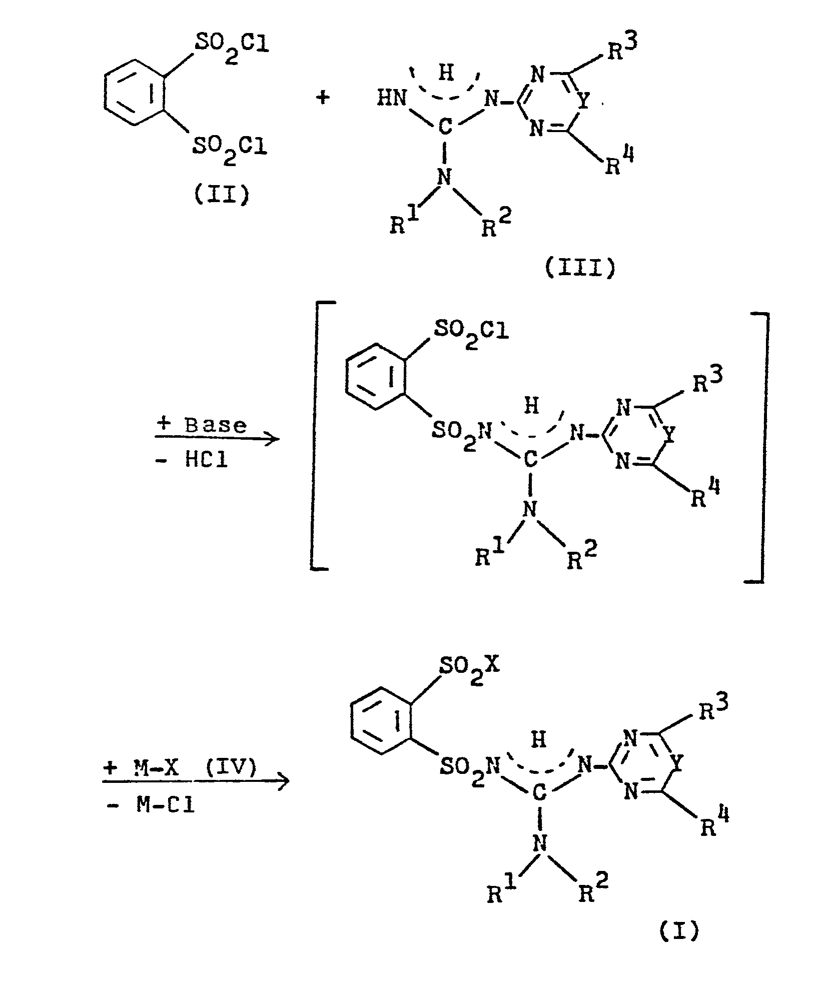

(In den Formeln haben R¹, R², R3, R⁴, Y, _{X} und _{M} die im Vorstehenden angegebenen Bedeutungen).

In dem vorstehenden Reaktionsschema bezeichnet X eine niedere Alkoxy-Gruppe, eine niedere Alkylamino-Gruppe oder eine Morpholino-Gruppe. Spezielle Beispiele für die niedere Alkoxy-Gruppe sind solche, die niedere Alkyl-Gruppen wie Methyl, Ethyl, Propyl, Isopropyl und n- (iso, sec- oder tert-) -Butyl enthalten. Spezielle Beispiel für die niedere Alkylamino-Gruppe können solche mit den gleichen niederen Alkyl-Gruppen wie oben beispielhaft angegeben sein.
_{R}¹ bezeichnet ein Wasserstoff-Atom oder die Gruppe der Formel 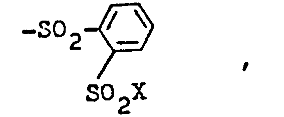
in der X die oben angegebene Bedeutung hat,
R² bezeichnet eine Hydroxy-Gruppe, eine niedere Alkoxy-Gruppe oder eine Di-Niederalkylamino-Gruppe. Beispiele für die niedere Alkoxy-Gruppe sind die gleichen wie oben angegeben. Beispiele für die Di-Niederalkylamino-Gruppe sind Amino-Gruppen, die durch die gleichen niederen Alkyl-Gruppen wie oben angegeben di-substituiert sind. Die niederen Alkyl-Gruppen können identisch oder verschieden sein.
_{R}³ und _{R}⁴ stehen jeweils für eine niedere Alkyl-Gruppe oder eine niedere Alkoxy-Gruppe. Beispiele für die niedere Alkyl-Gruppe oder die niedere Alkoxy-Gruppe sind die gleichen wie oben angegeben.
Y bezeichnet N oder CH.
M steht für ein Wasserstoff-Atom oder ein Alkalimetall-Atom wie Natrium, Kalium und Lithium.

Zu speziellen Beispielen der Ausgangs-Verbindung der allgemeinen Formel (III) in dem vorstehendn Reaktionsschema zählen
N-(4,6-Dimethylpyrimidin-2-yl)-N'-methoxyguanidin, N-(4,6-Dimethoxy-1,3,5-triazin-2-yl)-N'-methoxy- guanidin,
N-(4-Methoxy-6-methylpyrimidin-2-yl)-N'-methoxy- guanidin,
N-(4,6-Dimethylpyrimidin-2-yl)-N'-hydroxyguanidin, N-(4,6-Dimethylpyrimidin-2-yl)-N'-methoxy-N'-(2- ethoxysulfonylbenzolsulfonyl)guanidin und N-(4,6-Dimethylpyrimidin-2-yl)-N'-dimethylamino- guanidin.

Zu speziellen Beispielen für die Verbindung der allgemeinen Formel (IV), die in gleicher Weise ein Ausgangsstoff ist, zählen
Natriummethylat,
Natriumethylat,
Ammoniak,
Methylamin und
Morpholin.

Pyridin ist ein Beispiel für die in der vorstehenden Reaktion verwendete Base.

Das Verfahren i) wird anhand des folgenden typischen Beispiels speziell beschrieben. 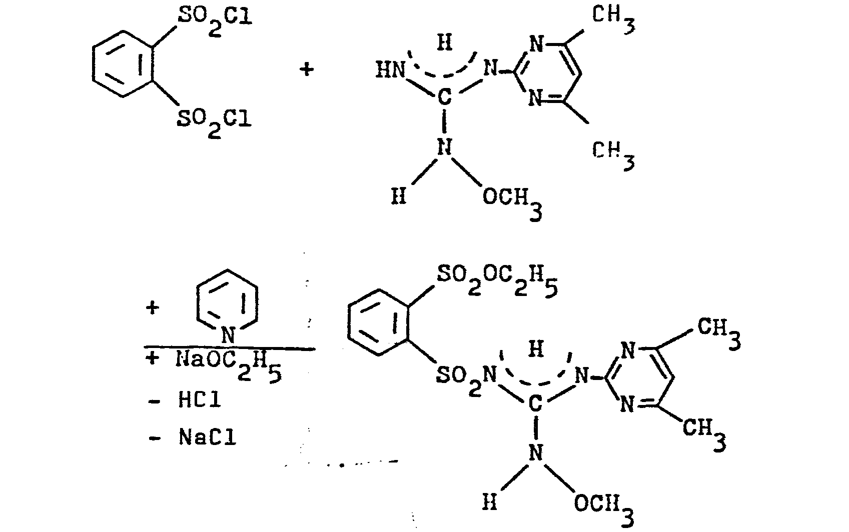 Das vorstehende Verfahren zur Herstellung der Verbindungen der vorliegenden Erfindung kann zweckmäßigerweise unter Verwendung eines Lösungsmittels oder Verdünnungsmittels durchgeführt werden. Zu diesem Zweck können sämtliche inerten Lösungsmittel und Verdünnungsmittel verwendet werden.

Beispiele für solche Lösungsmittel oder Verdünnungsmittel umfassen Wasser; aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Di-isopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Alkohole wie Methanol, Ethanol, Isopropanol, Butanol und Ethylenglycol; Ester wie Ethylacetat und Amylacetat; Säureamide wie Dimethylformamid und Dimethylacetamid; Sulfone und Sulfoxide wie Dimethylsulfoxid und Sulfolan; sowie Basen wie Pyridin.

Die obige Reaktion kann in Gegenwart eines säurebindenden Mittels durchgeführt werden. Beispiele für das säurebindende Mittel umfassen die Hydroxide, Carbonate, Hydrogencarbonate und Alkoholate von Alkalimetallen, sowie tertiäre Amine wie Triethylamin, Diethylanilin und Pyridin .

Das Verfahren gemäß der vorliegenden Erfindung kann in einem weiten Temperaturbereich durchgeführt werden. Im allgemeinen kann es bei einer Temperatur zwischen etwa -20°C und dem Siedepunkt der Mischung, zweckmäßigerweise zwischen etwa 0°C und etwa 100°C, durchgeführt werden. Die Reaktion wird zweckmäßigerweise unter normalem Atmosphärendruck durchgeführt, jedoch ist es auch möglich, unter erhöhtem oder vermindertem Druck zu arbeiten.

### Verfahren ii)

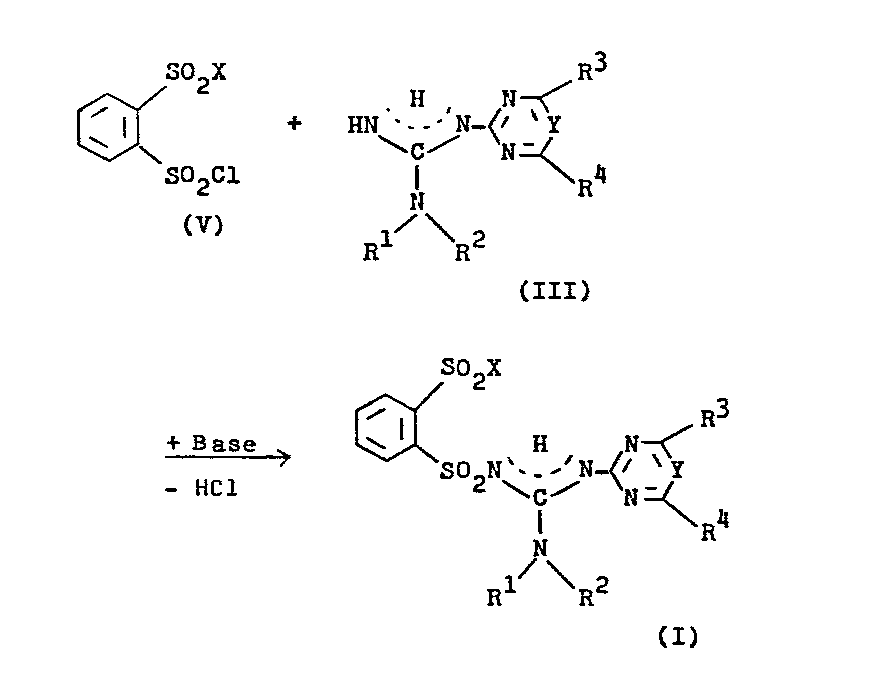

In den vorstehenden Formeln haben R , _{R}², R³, _{R}⁴, _{Y} und X die im Vorstehenden angegebenen Bedeutungen. In dem vorstehenden Reaktionsschema haben R , R², R³, R , Y und X die gleichen Bedeutungen, wie sie im Vorstehenden in bezug auf das Verfahren i) angegeben sind.

Zu speziellen Beispielen der Ausgangs-Verbindung der allgemeinen Formel (V) zählen
2-Methoxysulfonylbenzolsulfonylchlorid, 2-Ethoxysulfonylbenzolsulfonylchlorid, 2-Morpholinosulfonylbenzolsulfonylchlorid, 2-Methylaminosulfonylbenzolsulfonylchlorid und 2-Aminosulfonylbenzolsulfonylchlorid.

Spezielle Beispiele für die andere Ausgangs-Verbindung der allgemeinen Formel (III) sind die gleichen wie diejenigen, die oben in bezug auf das Verfahren i) angegeben sind.

Spezielle Beispiele für die Base sind die gleichen wie die oben in bezug auf das Verfahren i) angegebenen.

Das Verfahren ii) wird anhand des folgenden typischen Beispiels speziell beschrieben. 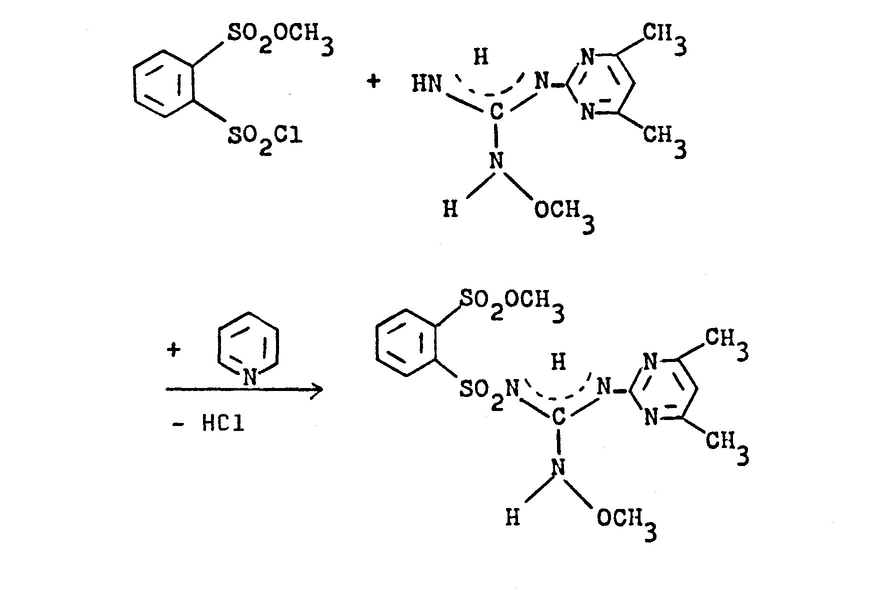 Zweckmäßigerweise wird das Verfahren ii) unter Verwendung eines derjenigen Lösungsmittel oder Verdünnungsmittel durchgeführt, wie sie oben beispielhaft genannt sind, und das gewünschte Endprodukt mit hoher Reinheit kann in hoher Ausbeute gewonnen werden.

Wie dies bei dem Verfahren i) der Fall ist, kann auch das Verfahren ii) in einem breiten Temperaturbereich durchgeführt werden. Zweckmäßigerweise wird die Reaktion unter normalem Atmosphärendruck durchgeführt, jedoch ist es auch möglich, unter erhöhtem oder vermindertem Druck zu arbeiten.

### Verfahren iii)

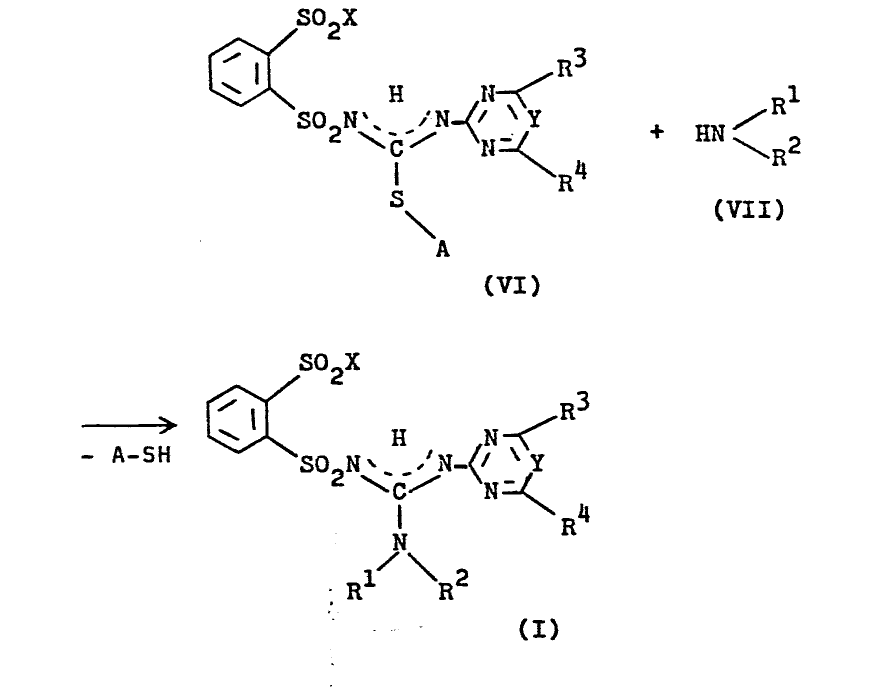

In den vorstehenden Formeln haben _{R1}, _{R}², _{R}³, _{R}⁴, _{Y}, _{X} und A die im Vorstehenden angegebenen Bedeutungen. In dem vorstehenden Reaktionsschema haben R , R², R , R⁴, Y und X die gleichen Bedeutungen, wie sie im Vorstehenden angegeben sind. A bezeichnet eine niedere Alkyl-Gruppe, wobei spezielle Beispiele für diese die gleichen sind, wie sie oben in bezug auf das Verfahren i) angegeben sind.

Zu speziellen Beispielen der Verbindung der allgemeinen Formel (VI) als Ausgangsstoff in dem vorstehenden Reaktionsschema zählen
N-(4,6-Dimethylpyrimidin-2-yl)-N'-(2-methoxysulfo- nylbenzolsulfonyl)-S-methylisothioharnstoff, N-(4,6-Dimethylpyrimidin-2-yl)-N'-(2-ethoxysulfo- nylbenzolsulfonyl)-S-methylisothioharnstoff, N-(4,6-Dimethylpyrimidin-2-yl)-N'-(2-morpholino- sulfonylbenzolsulfonyl)-S-methylisothioharn- stoff, N-(4,6-Dimethylpyrimidin-2-yl)-N'-(2-methylamino- sulfonylbenzolsulfonyl)-S-methylisothioharn- stoff, N-(4,6-Dimethylpyrimidin-2-yl)-N'-(2-aminosulfo- nylbenzolsulfonyl)-S-methylisothioharnstoff und N-(4,6-Dimethoxy-1,3,5-triazin-2-yl)-N'-(2-meth- oxysulfonylbenzolsulfonyl)-S-methylisothio- harnstoff.

Zu speziellen Beispielen für die Ausgangs-Verbindung der allgemeinen Formel (VII) zählen O-Methylhydroxylamin, O-Ethylhydroxylamin, Hydroxylamin, N-Methoxy-2-ethoxysulfonylbenzolsulfonamid und N,N-Dimethylhydrazin.

Das vorstehende Verfahren iii) wird anhand des folgenden typischen Beispiels speziell beschrieben.

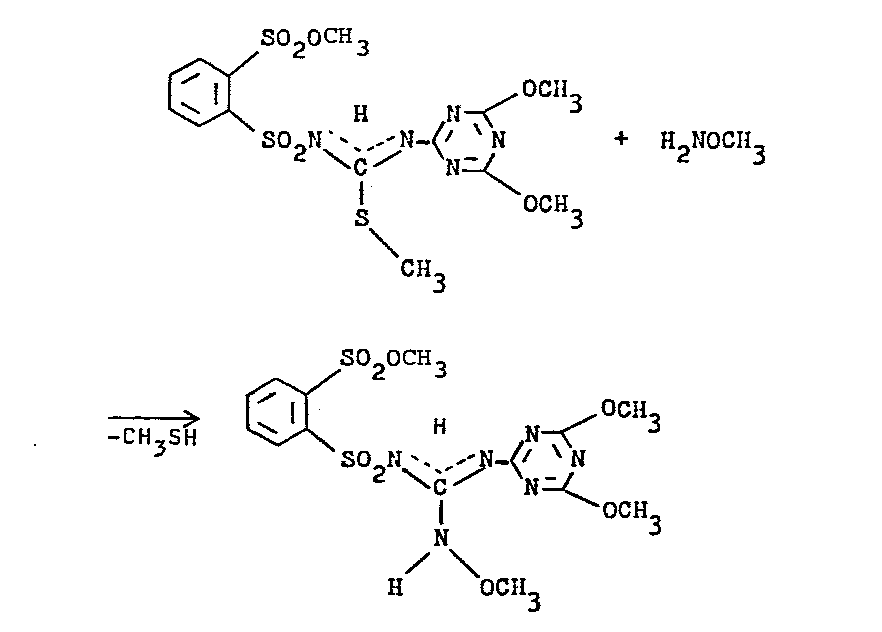

Zweckmäßigerweise wird das Verfahren iii) unter Verwendung eines derjenigen Lösungsmittel oder Verdünnungsmittel durchgeführt, wie sie oben beispielhaft genannt sind, und das gewünschte Endprodukt mit hoher Reinheit kann in hoher Ausbeute gewonnen werden.

Wie dies bei den Verfahren i) und ii) der Fall ist, kann auch das Verfahren iii) in einem breiten Temperaturbereich durchgeführt werden. Zweckmäßigerweise wird die Reaktion unter normalem Atmosphärendruck durchgeführt, jedoch ist es auch möglich, unter erhöhtem oder vermindertem Druck zu arbeiten.

Die Verbindungen gemäß der vorliegenden Erfindung können in einfacher Weise mittels der oben beschriebenen Verfahren i, ii) und iii) hergestellt werden. Von diesen Verbindungen können N,N'-Bis(2-substituierte Benzolsulfonyl)-Guanidin-Derivate, 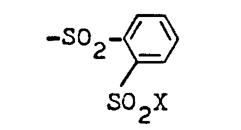 in denen die Gruppe an jedem der beiden

Stickstoff-Atome des Guanidin-Skeletts substituiert ist, auch mittels der folgenden Alternativ-Verfahren hergestellt werden.

### Alternativ-Verfahren a)

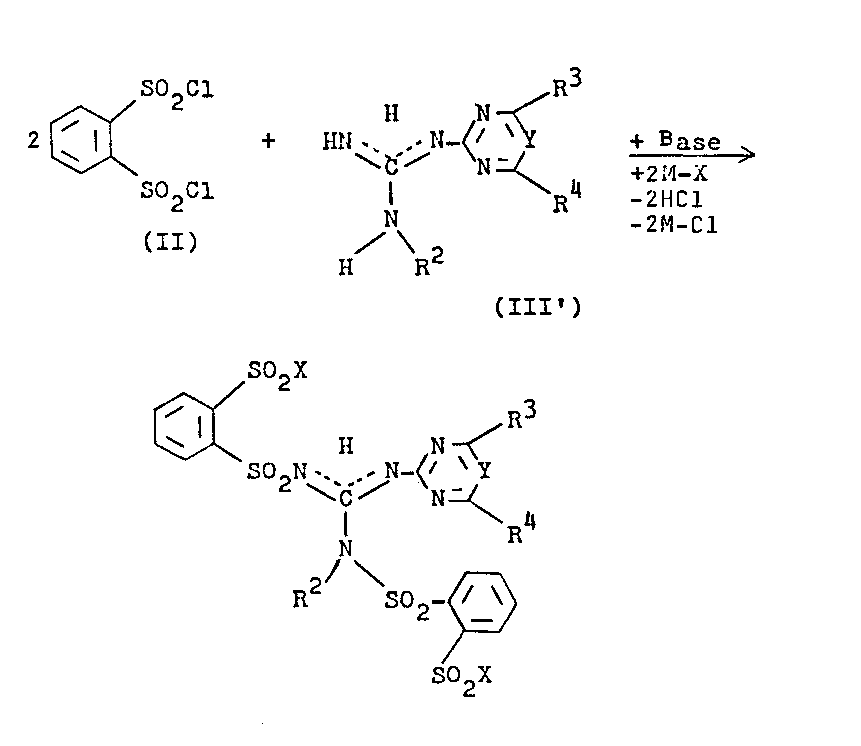

Alternativ-Verfahren b) 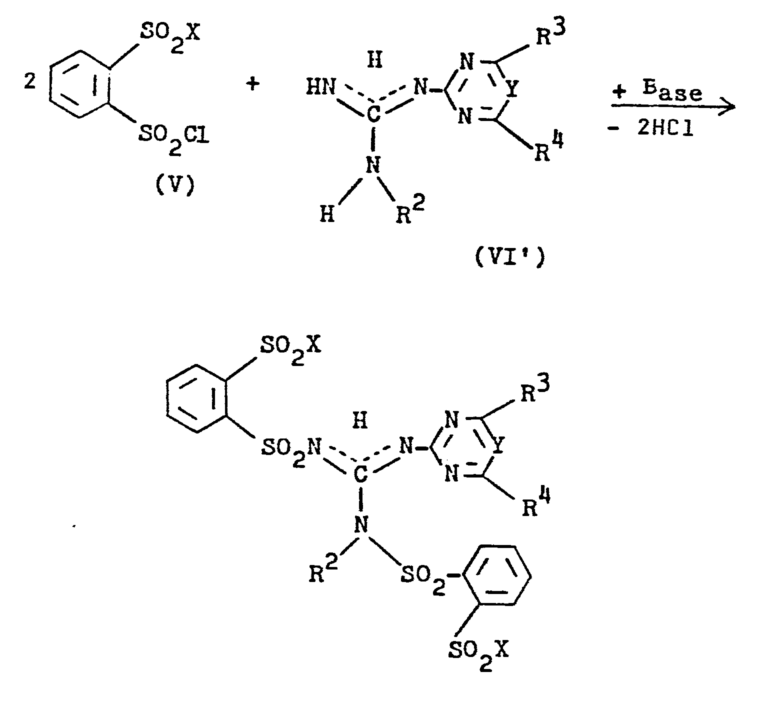 Mittels der Alternativ-Verfahren a) und b) können die gewünschten Verbindungen gemäß der vorliegenden Erfindung in wirksamer Weise dadurch hergestellt werden, daß 1 mol der Ausgangs-Verbindungen der allgemeinen Formeln (II) und (V) mit etwa 2 bis etwa 2,5 mol der anderen Ausgangs-Verbindungen (III') bzw. (VI') umgesetzt werden.

Typische Beispiele für die Alternativ-Verfahren sind nachstehend angegeben. 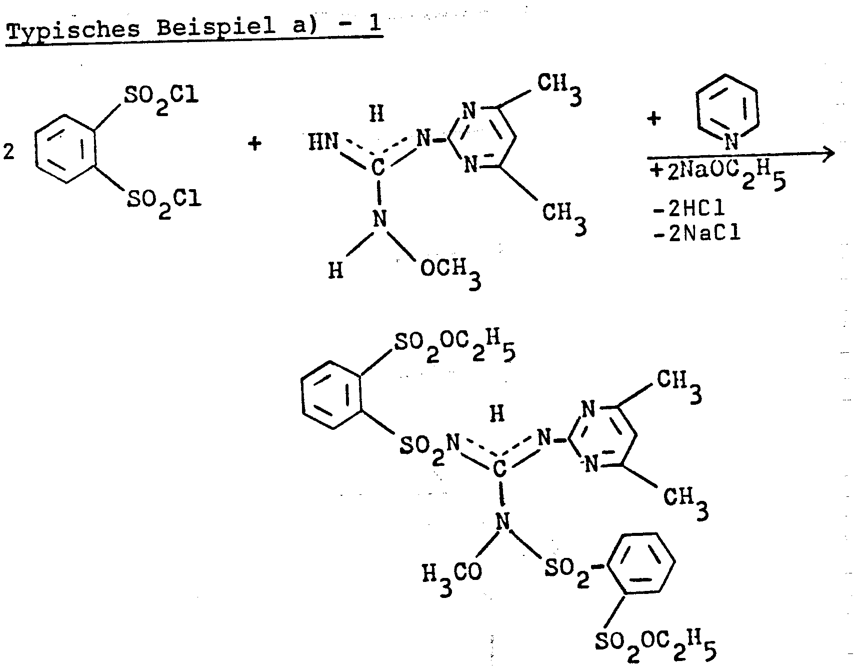 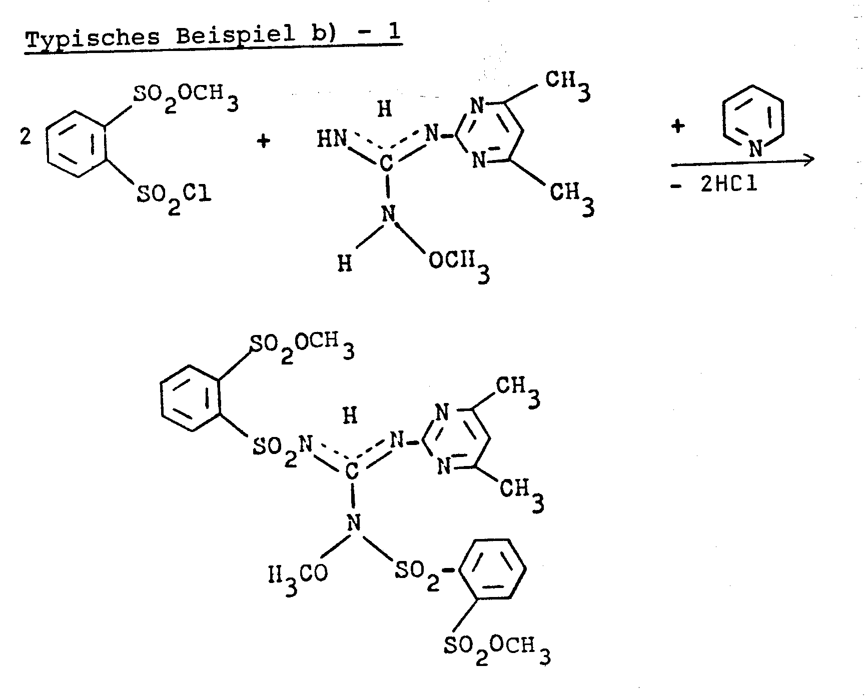 Die vorstehenden Verfahren können unter Verwendung derjenigen Lösungsmittel oder Verdünnungsmittel durchgeführt werden, wie sie oben beispielhaft genannt sind, wonach die Endprodukte mit hoher Reinheit und in hoher Ausbeute erhalten werden. Die Reaktionsbedingungen können die gleichen wie diejenigen sein, die oben in bezug auf das Verfahren i) angegeben sind.

Als Herbizide können die Verbindungen der allgemeinen Formel (I) gemäß der vorliegenden Erfindung als solche unmittelbar nach dem Verdünnen mit Wasser oder in Form verschiedenartiger Präparate, wie sie gewöhnlich mittels der in der Herstellung von Agrochemikalien üblichen Verfahren unter Verwendung von landwirtschaftlich unbedenklichen Hilfsstoffen erhalten werden, angewandt werden. Beim praktischen Einsatz werden diese verschiedenartigen Präparate entweder unmittelbar oder nach dem Verdünnen mit Wasser auf die gewünschten Konzentrationen gebracht.

Beispiele für die agrochemisch unbedenklichen Hilfsstoffe, auf die hier Bezug genommen wird, umfassen beispielsweise Verdünnungsmittel (Lösungsmittel, Streckmittel, Träger), oberflächenaktive Stoffe (Lösungsvermittler, Emulgatoren, Dispergiermittel, Netzmittel), Stabilisatoren, Haftmittel, Aerosol-Treibmittel und Synergisten.

Beispiele für die Lösungsmittel sind Wasser und organische Lösungsmittel, beispielsweise Kohlenwasserstoffe L-Z.B. n-Hexan, Petrolether, Erdöl-Fraktionen (z.B. Paraffinwachse, Kerosin, Leichtöle, Mittelöle und Schweröle), Benzol, Toluol und Xylol_7, halogenierte Kohlenwasserstoffe (z.B. Methylenchlorid, Kohlenstofftetrachlorid, Ethylenchlorid, Ethylendibromid, Chlorbenzol und Chloroform), Alkohole (z.B. Methylalkohol, Ethylalkohol, Propylalkohol und Ethylenglycol), Ether (z.B. Diethylether, Ethylenoxid und Dioxan), Alkoholether (z.B. Ethylenglycol-monomethylether), Ketone (z.B. Aceton und Isophoron), Ester (z.B. Ethylacetat und Amylacetat), Amide (z.B. Dimethylformamid und Dimethylacetamid) und Sulfoxide (z.B. Dimethylsulfoxid).

Beispiele für die Streckmittel oder Träger umfassen anorganische Pulver, beispielsweise Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z.B. Pyrophyllit, Talkum, Montmorillonit, Beidellit, Vermikulit, Kaolinit und Glimmer), pflanzliche Pulver wie beispielsweise Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für die oberflächenaktiven Mittel umfassen anionische oberflächenaktive Mittel wie Alkylsulfonsäureester (z.B. Natriumlaurylsulfat), Arylsulfonsäure-Salze (z.B. Alkylarylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z.B. Laurylamin, Stearyltrimethylammoniumchlorid und Alkyldimethylbenzylammoniumchlorid) und Polyoxyethylenalkylamine, nicht-ionische oberflächenaktive Mittel wie Polyoxyethylenglycolether (z.B. Polyoxyethylenalkylarylether und deren Kondensationsprodukte), Polyoxyethylenglycolester (z.B. Polyoxyethylenfettsäureester) und Ester mehrwertiger Alkohole (z.B. Polyoxyethylensorbitan-monolaurat) sowie amphotere oberflächenaktive Mittel.

Beispiele für andere Hilfsstoffe umfassen Stabilisatoren, Haftmittel (z.B. landwirtschaftliche Seifen, Casein-Kalk, Natriumalginat, Polyvinylalkohol, Haftmittel vom Vinylacetat-Typ und Acryl-Haftmittel), wirkungsverlängernde Mittel, Dispersions-Stabilisatoren (z.B. Casein, Tragant, Carboxymethylcellulose (CMC) und Polyvinylalkohol (PVA)) und synergistische Mittel.

Die Verbindungen der vorliegenden Erfindung können mittels der allgemein auf dem Gebiet der Agrochemikalien gebräuchlichen Verfahren zu verschiedenen Präparaten formuliert werden. Beispiele für solche Anwendungsformen sind emulgierbare Konzentrate, öl-Präparate, benetzbare Pulver, lösliche Pulver, Suspensionen, Stäubemittel, Granulat und pulvrige Präparate.

Die Herbizide gemäß der vorliegenden Erfindung können von etwa 0,001 bis etwa 100 Gew.-%, vorzugsweise etwa von 0,005 bis etwa 95 Gew.-%, der vorerwähnten Wirkstoffe enthalten.

Für den praktischen Gebrauch beträgt die geeignete Menge der aktiven Verbindung in den vorgenannten Mitteln in ihren verschiedenen Formen und gebrauchsfertigen Präparaten beispielsweise etwa 0,01 bis etwa 95 Gew.-%, vorzugsweise etwa 0,05 bis etwa 60 Gew.-%. Der Gehalt des Wirkstoffs kann in geeigneter Weise je nach der Art des Präparats, dem Verfahren, Zweck, der Zeit und dem Ort seiner Anwendung, dem Zustand des Auftretens der Unkräuter etc. variiert werden.

Erforderlichenfalls können die Verbindungen der allgemeinen Formel (I) gemäß der vorliegenden Erfindung weiterhin auch in Kombination mit anderen Agrochemikalien verwendet werden, beispielsweise mit Insektiziden, Fungiziden, Mitiziden, Nematiziden, Anti-Virus-Mitteln, anderen Herbiziden, Pflanzen-Wachstumsregulatoren und Lockstoffen / z.B. Organophosphat-Verbindungen, Carbamat-Verbindungen, Dithio(oder thiol)carbamat-Verbindungen, organischen Chlor-Verbindungen, Dinitro-Verbindungen, organischen Schwefel- oder metallorganischen Verbindungen, Antibiotika, substituierten Diphenylether-Verbindungen, Harnstoff-Verbindungen und TriazinVerbindungen_7 und/oder Düngemitteln.

Die den im Vorstehenden bezeichneten Wirkstoff enthaltenden verschiedenartigen Mittel und gebrauchsfertigen Präparate können mittels verschiedener Verfahren zur Anwendung gebracht werden, wie sie allgemein für das Aufbringen von Agrochemikalien gebräuchlich sind, beispielsweise durch Sprühen (Versprühen von Flüssigkeiten, Vernebeln, Zerstäuben, Stäuben, Streuen von Granulat, Wasser-Oberflächenbehandlung, Gießen etc.) und Bodenbehandlung (Vermischen mit dem Boden, Streuen etc.). Sie können auch mittels des sogenannten Ultra-Low-Volume-Sprühverfahrens aufgebracht werden. Nach diesem Verfahren kann der Wirkstoff sogar in einer Konzentration von 100 % zur Anwendung gelangen.

Die Aufwandmenge pro Flächeneinheit beträgt beispielsweise etwa 0,01 bis etwa 3 kg/ha, vorzugsweise etwa 0,025 bis etwa 1 kg/ha. In speziellen Fällen können jedoch oder sollten sogar die Aufwandmengen außerhalb des angegebenen Bereichs liegen.

Gemäß der vorliegenden Erfindung kann ein herbizides Mittel zur Verfügung gestellt werden, das als Wirkstoff eine Verbindung der allgemeinen Formel (I) sowie ein Verdünnungsmittel (ein Lösungsmittel und/oder ein Streckmittel und/oder einen Träger) und/oder ein oberflächenaktives Mittel und, falls weiterhin erforderlich, einen Stabilisator, ein Haftmittel, ein synergistisches Mittel etc. enthält.

Die vorliegende Erfindung macht weiterhin ein Verfahren zur Unkrautbekämpfung verfügbar, das darin besteht, daß eine Verbindung der allgemeinen Formel (I) allein oder in Form einer Mischung mit einem Verdünnungsmittel (einem Lösungsmittel und/oder einem Streckmittel und/ oder einem Träger) und/oder einem oberflächenaktiven Mittel und, falls weiterhin erforderlich, einem Stabilisator, einem Haftmittel, einem synergistischen Mittel etc. auf Unkräuter und/oder ihren Lebensraum aufgebracht wird.

Die vorliegende Erfindung wird durch die folgenden Beispiele näher erläutert, ist jedoch nicht auf diese speziellen Beispiele allein beschränkt.

### Beispiel 1

N-(4,6-Dimethylpyrimidin-2-yI)-N'-methoxyguanidin (9,75 g) wurde in Pyridin (200 ml) gelöst, und 1,2-Benzoldisulfonylchlorid (13,75 g) wurde zu der Lösung hinzugefügt, und die Lösung wurde 2 Tage bei Raumtemperatur gerührt. Nach der Reaktion wurde das Pyridin unter vermindertem Druck abgedampft. Das zurückbleibende öl wurde mit Methylenchlorid extrahiert. Die organische Schicht wurde mit 1 N wäßriger Salzsäure gewaschen und über Natriumsulfat getrocknet. Das Methylenchlorid wurde unter vermindertem Druck abgedampft.

Der Rückstand wurde mit Natriummethylat (0.05 mol) in Methanol 2 h auf 50°C erhitzt. Die Reaktionsmischung wurde in Wasser gegossen, die wäßrige alkalische Lösung wurde mit Salzsäure neutralisiert, und der Rückstand wurde durch Filtration gesammelt. Umkristallisation des Rückstands aus Methanol lieferte 10 g des gewünschten N-(4,6-Dimethylpyrimidin-2-yl)-N'-(2-methoxysulfonyl- benzolsulfonyl)-N"-methoxyguanidins der nachstehenden Formel. Schmp. 130-138°C.

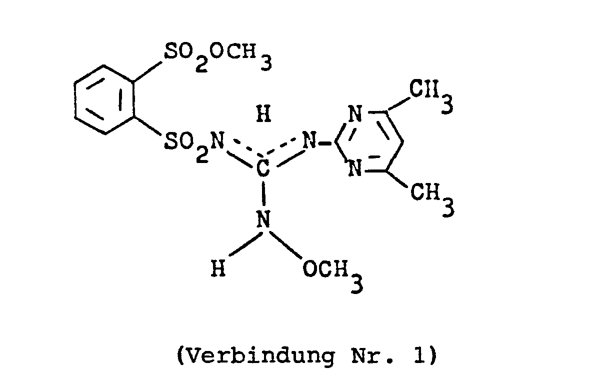

### Beispiel 2

N-(4,6-Dimethylpyrimidin-2-yl)-N'-methoxyguanidin (9,75 g) wurde in Pyridin (200 ml) gelöst, und 2-Methoxysulfonylbenzolsulfonylchlorid (13,5 g) wurde zu der Lösung hinzugefügt. Die Mischung wurde 2 Tage bei Raumtemperatur gerührt. Die Reaktionsmischung wurde in Wasser gegossen und mit Methylenchlorid extrahiert. Die organische Schicht wurde mit einer 1 N wäßrigen Salzsäure-Lösung gewaschen und über wasserfreiem Natriumsulfat getrocknet. Das Methylenchlorid wurde unter reduziertem Druck abgedampft, und der Rückstand wurde aus Methanol umkristallisiert, wonach 10 g des gewünschten N-(4,6-Dimethylpyrimidin-2-yl)-N'-(2-methoxysulfonyl- benzolsulfonyl)-N"-methoxyguanidins der nachstehenden Formel erhalten wurden. Schmp. 130-138°C.

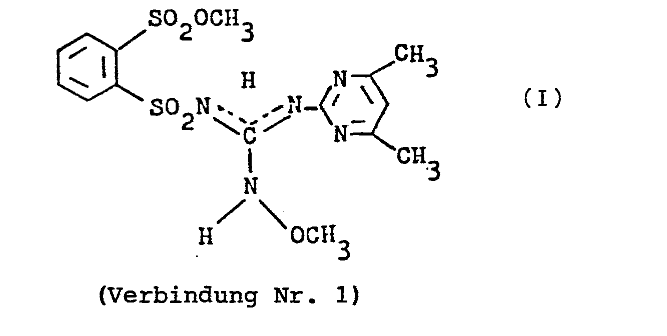

### Beispiel 3

N-(4,6-Dimethoxy-1,3,5-triazin-2-yl)-N'-(2-methoxysul- fonylbenzolsulfonyl)-S-methylisothioharnstoff (4,63 g) wurde in Dioxan (100 ml) gelöst, und O-Methylhydroxylamin (2,35 g) wurde zu der Lösung hinzugefügt. Die Mischung wurde 4 Tage bei Raumtemperatur gerührt. Die Reaktionsmischung wurde in Eiswasser gegossen und mit Salzsäure auf pH 3 eingestellt. Die ausgefallenen rohen Kristalle wurden durch Filtration gesammelt und aus Acetonitril umkristallisiert, wonach 1,5 g des gewünschten N-(4,6-Dimethoxy-1,3,5-triazin-2-yl)-N'-(2-methoxysulfonylbenzolsulfonyl)-N"-methoxyguanidins der nachstehenden Formel erhalten wurden. Schmp. 148-150°C.

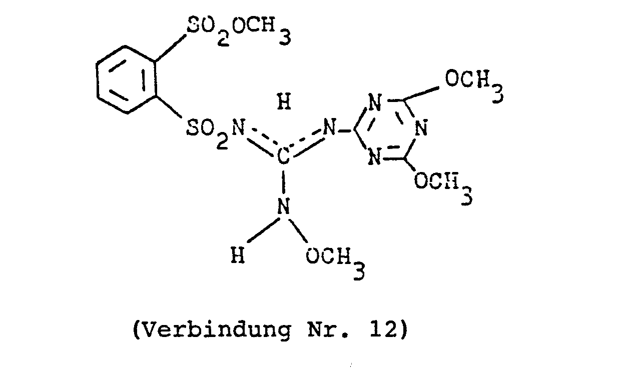

Nach den gleichen Arbeitsweisen wie in Beispiel 1 oder 2 wurden die in der nachstehenden Tabelle 1 aufgeführten erfindungsgemäßen Verbindungen synthetisiert.

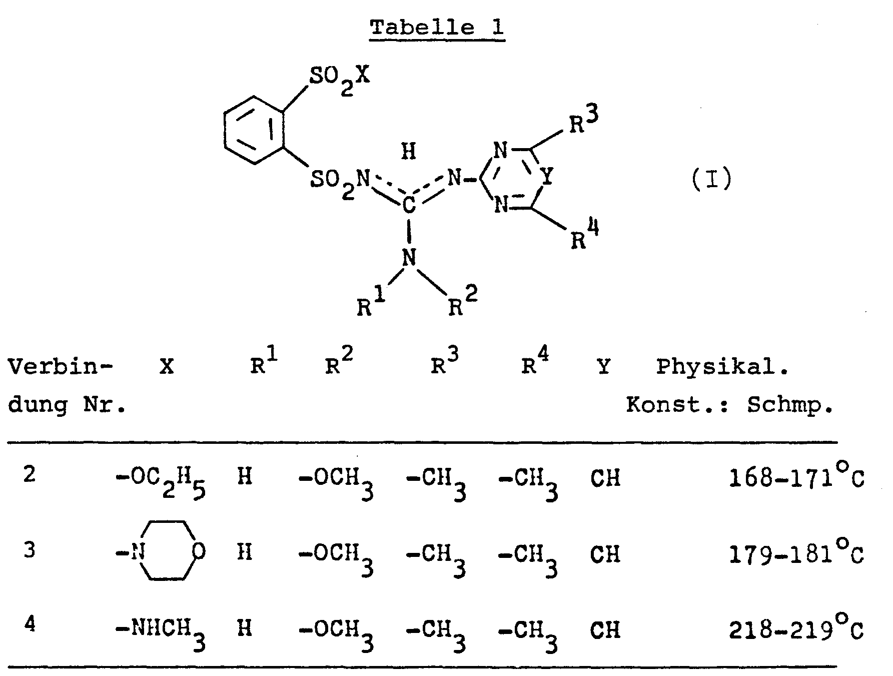

### Beispiel 4

Die folgende Tabelle 2 zeigt erfindungsgemäße Verbindungen, die nach den gleichen Arbeitsweisen wie in Beispiel 1 oder 2 unter Verwendung der speziell in Tabelle 2 dargestellten Stoffe synthetisiert wurden.

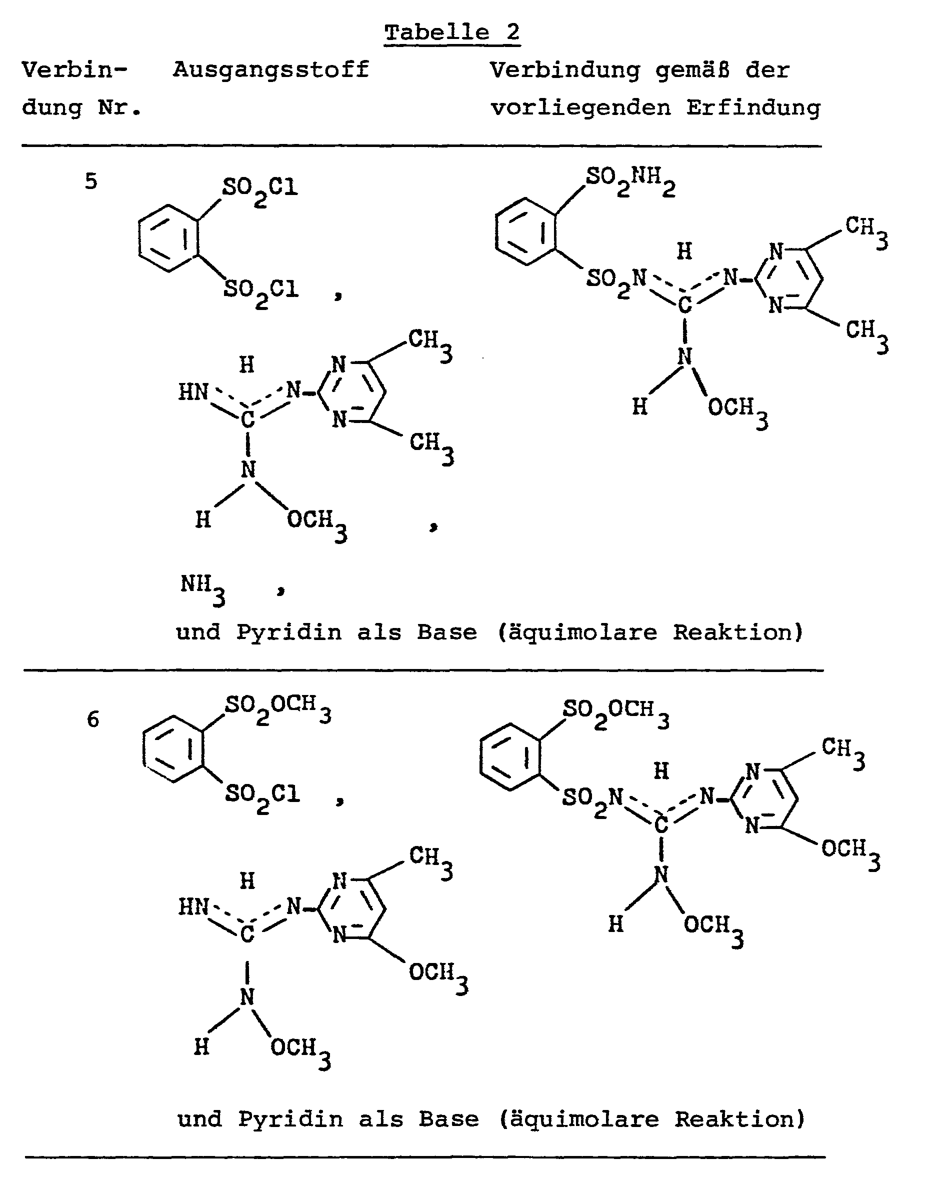

### Beispiel 5

Die nachstehende Tabelle 3 zeigt die Verbindungen gemäß der vorliegenden Erfindung, die nach den Alternativ-Verfahren a) oder b) unter Verwendung der in Tabelle 3 dargestellten Ausgangsstoffe synthetisiert wurden.

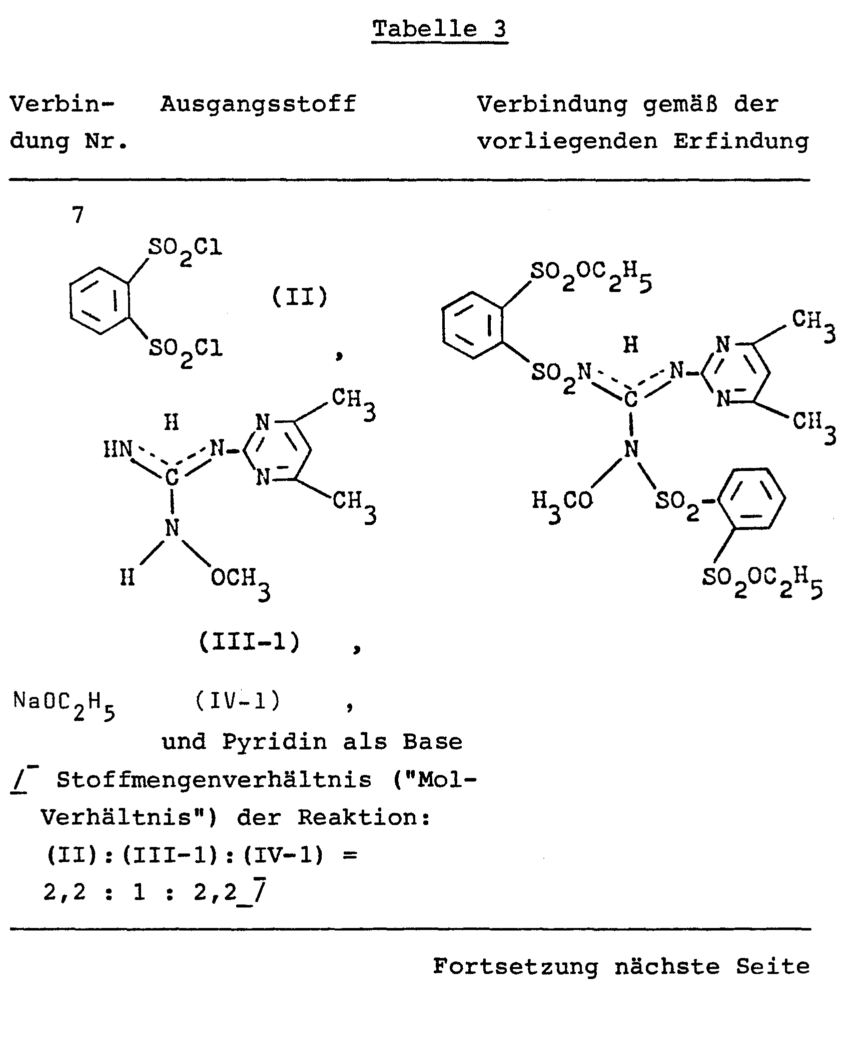

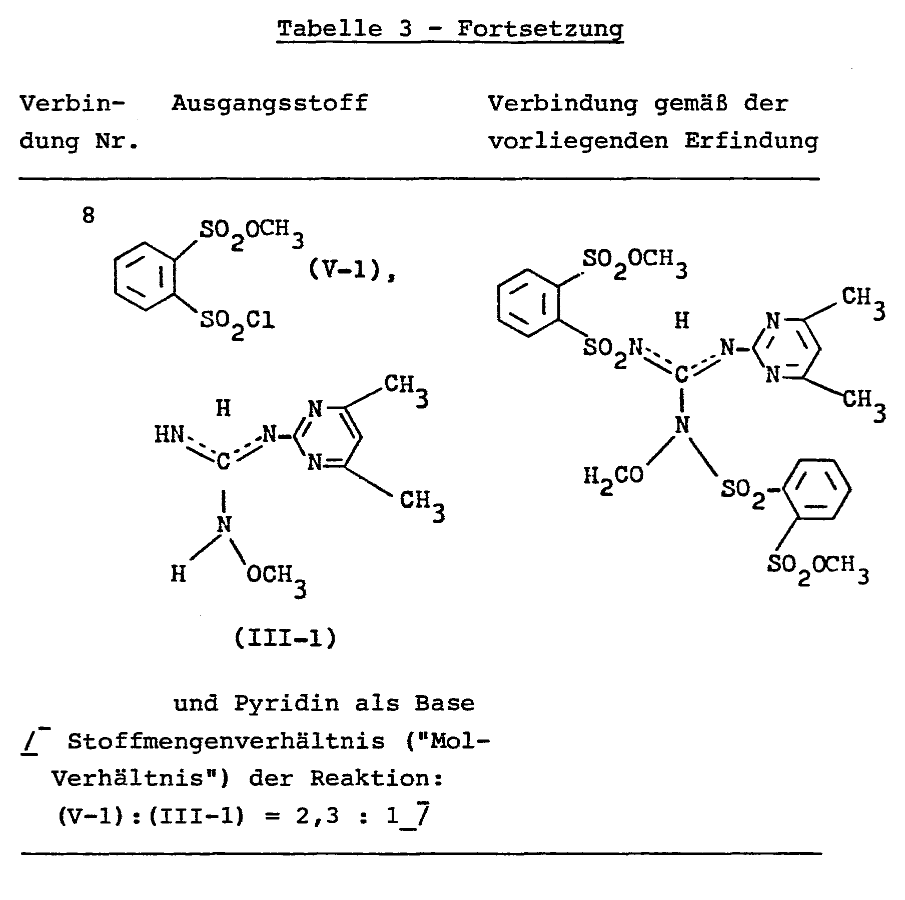

### Beispiel 6

Die Verbindungen gemäß der vorliegenden Erfindung können in Form von Alkalimetall-Salzen vorliegen. Spezielle Beispiele für solche synthetisierten Verbindungen sind in der Tabelle 4 aufgeführt.

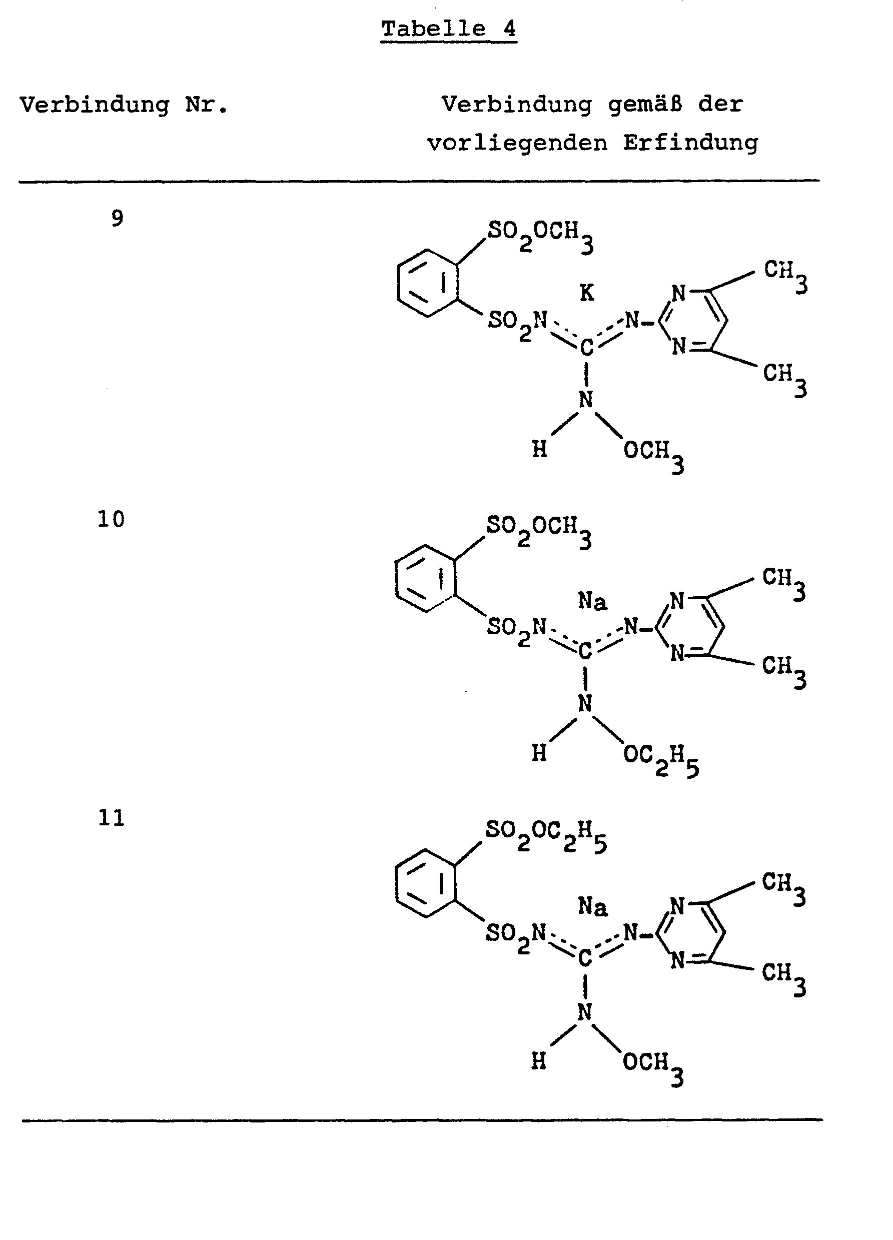

### Beispiel 7

### Benetzbares Pulver.

15 Teile der Verbindung Nr. 1 der Erfindung, 80 Teile eines Gemisches (1:5) aus pulvriger Diatomeenerde und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf Unkräuter und/oder ihren Lebensraum aufgetropft.

### Beispiel 8

### Emulgierbares Konzentrat.

30 Teile der Verbindung Nr. 2 der Erfindung, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf Unkräuter und/oder ihren Lebensraum aufgetropft.

### Beispiel 9

### Stäubemittel.

2 Teile der Verbindung Nr. 3 der Erfindung und 98 Teile Tonpulver wurden pulverisiert und gemischt, wodurch ein Stäubemittel hergestellt wird. Dieses wird über Unkräutern und/oder ihrem Lebensraum ausgestreut.

### Beispiel 10

### Stäubemittel.

Die Verbindung Nr. 4 der Erfindung (1,5 Teile), 0,5 Teile Isopropylhydrogenphosphat (PAP) und 98 Teile Tonpulver werden pulverisiert und gemischt, wodurch ein Stäubemittel hergestellt wird. Dieses wird über Unkräutern und/oder ihrem Lebensraum ausgestreut.

### Beispiel 11

### Granulat.

Wasser (25 Teile) wird zu einer Mischung aus 10 Teilen der Verbindung Nr. 1 der Erfindung, 30 Teilen Bentonit (Montmorillonit), 58 Teilen Talkum und 2 Teilen Ligninsulfonat zugesetzt, und das Gemisch wird gut geknetet. Die Mischung wird mittels eines Extruder-Granulators zu einem Granulat mit einer Korngröße von 0,43 bis 2,0 mm (10 bis 40 mesh) verarbeitet und bei 40°C bis 50°C getrocknet, wodurch ein Granulat gebildet wird. Dieses wird über Unkräutern und/oder ihrem Lebensraum ausgestreut.

### Beispiel 12

### Granulat.

Ein Drehmischer wird mit 95 Teilen Tonmineral-Teilchen mit einer Teilchengrößen-Verteilung im Bereich von 0,2 bis 2 mm beschickt, und unter Drehen des Mischers werden 5 Teile der Verbindung Nr. 2 der Erfindung zur gleichmäßigen Benetzung auf die Teilchen aufgesprüht, und die Teilchen werden getrocknet, wodurch ein Granulat gebildet wird. Dieses wird über Unkräutern und/oder ihrem Lebensraum ausgestreut.

### Beispiel 13 (Biologischer Test)

Prüfung der Wirkung gegen Acker-Unkräuter und Nutzpflanzen durch Behandlung des Laubs und des Bodens nach dem Auflaufen:
Wirkstoff-Präparat:
Träger: 5 Gewichtsteile Aceton;
Emulgator: 1 Gewichtsteil Benzyloxypolyglycolether.

Zur Herstellung eines Wirkstoff-Präparats wird 1 Gew.-Teil jeder der aktiven Verbindungen mit dem Träger und Emulgator in den oben bezeichneten Mengen vermischt, und eine vorher festgelegte Menge des resultierenden emulgierbaren Konzentrats wurde mit Wasser verdünnt.

Test-Verfahren:
Weizen-Saatgut wurde in mit Ackerboden gefüllte Töpfe (10 dm²) eingesät, und Erde, die Samen jeder der Species Alopecurus aequalis Sobol. var. amurensis Ohwi, Stellaria media Villars. und Stellaria alsine Grimn. enthielt, wurde auf den Boden aufgebracht, so daß sie diesen bis zu einer Tiefe von 1 cm bedeckte.

10 Tage nach dem Keimen (wenn der Weizen und die Unkrautarten sich im Zwei-Blatt-Stadium befanden),wurden 10 ml der Chemikalie in einer Konzentration von 25 ppm gleichmäßig auf die Oberflächenschicht des Bodens in jedem der Test-Töpfe aufgesprüht.

Vier Wochen nach dem Sprühen wurden die herbizide Wirkung und der Grad der Phytotoxizität gegenüber Weizen mit Hilfe einer Skala von 0 bis 5 nach dem folgenden Maßstab bewertet.

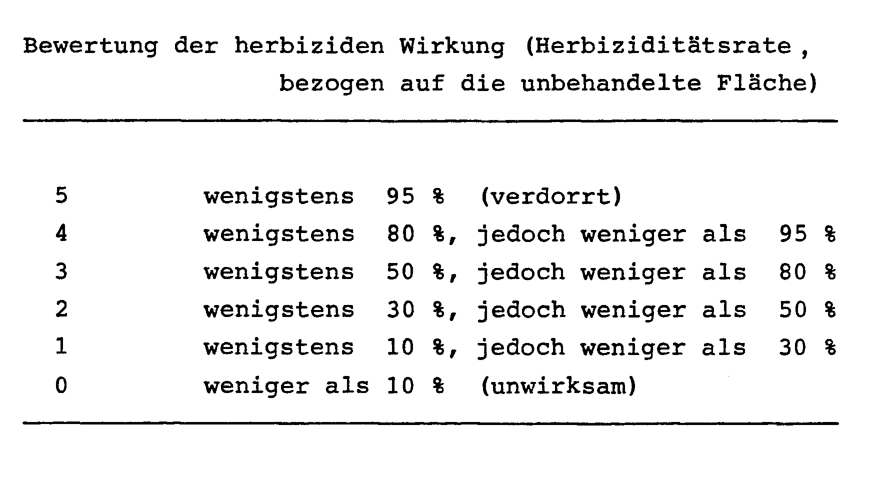

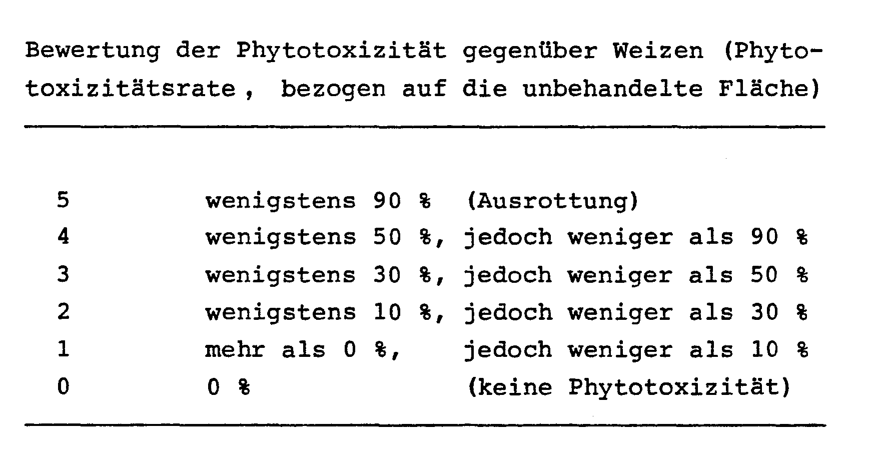

Die Ergebnisse sind in Tabelle 5 aufgeführt.

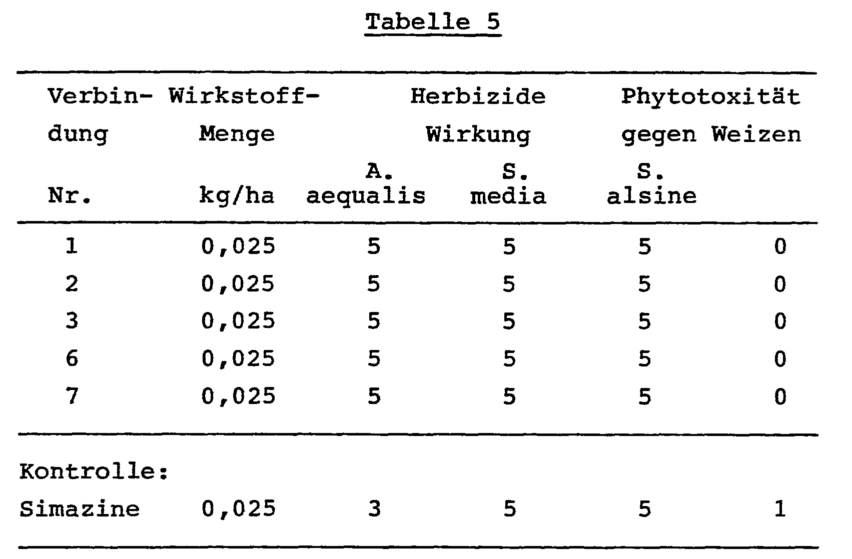

### Anmerkung:

Simazin (Kontrolle; Handelsprodukt): 2-Chloro-4,6-bis-(ethylamino)-1,3,5-triazin (50-proz. benetzbares Pulver).

### Beispiel 14 (Biologischer Test)

Prüfung der Wirkung gegen Acker-Unkräuter und Nutzpflanzen durch Vorauflauf-Behandlung des Bodens:
Test-Verfahren:
   Sojabohnen-Saatgut wurde in mit Ackerboden gefüllte Töpfe (10 dm^{z}) eingesät, und Erde, die Samen jeder der Species Digitaria adscendens Henr., Amaranthus lividus Loisel., Chenopodium album L.und Echinochloa crus-galli P.Beauv. enthielt, wurde auf den Boden aufgebracht, so daß sie diesen bis zu einer Tiefe von 1 cm bedeckte. Einen Tag nach dem Säen und Abdecken mit Erde wurden 10 ml einer Chemikalie mit einer Konzentration von 100 ppm, die in gleicher Weise wie in Beispiel 13 hergestellt worden war, gleichmäßig auf die Oberflächenschicht des Bodens in jedem der Test-Töpfe aufgesprüht.

Vier Wochen nach dem Sprühen wurden die herbizide Wirkung und der Grad der Phytotoxizität nach dem gleichen Maßstab wie in Beispiel 3 bewertet.

Die Ergebnisse sind in Tabelle 6 aufgeführt.

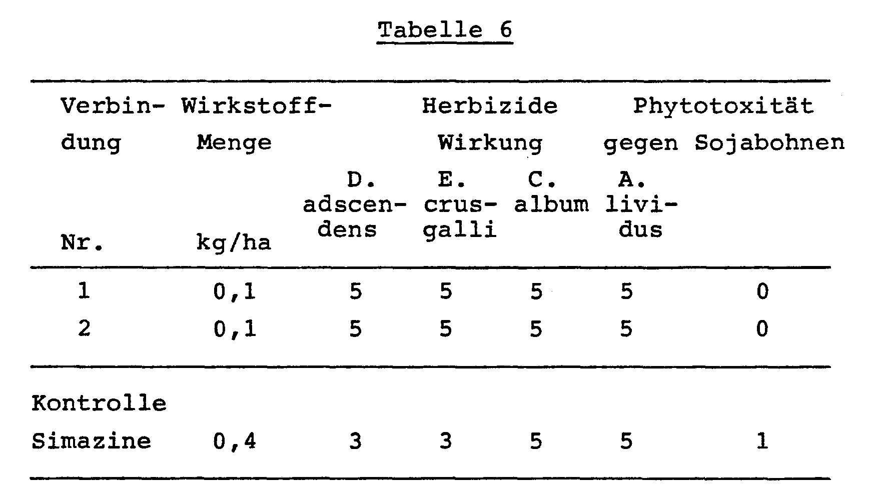

### Anmerkung:

Simazin (Kontrolle) ist die gleiche Verbindung, wie in der Fußnote zu Tabelle 5 angegeben.

## Patentansprüche

1. Sulfonylguanidin-Derivate der nachstehenden Formel 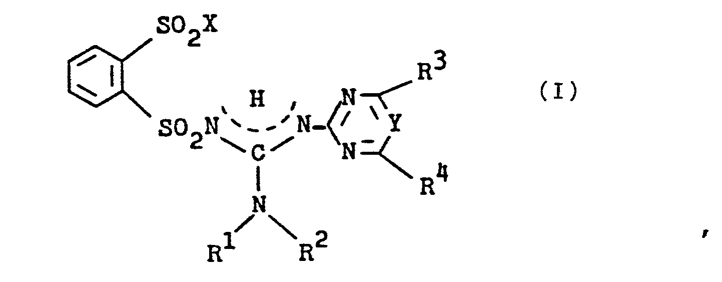 in der
X eine niedere Alkoxy-Gruppe, eine niedere Alkylamino-Gruppe oder eine Morpholino-Gruppe bezeichnet,
R¹ ein Wasserstoff-Atom oder die Gruppe 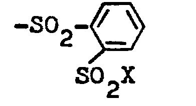 bezeichnet, in der X die oben angegebene Bedeutung hat,
R² eine Hydroxy-Gruppe, eine niedere Alkoxy-Gruppe, eine Di-Niederalkylamino-Gruppe bezeichnet,
_{R}³ und _{R}⁴ jeweils für eine niedere Alkyl-Gruppe oder eine niedere Alkoxy-Gruppe stehen und
Y N oder CH bezeichnet.

2. N-(2-Ethoxysulfonylbenzolsulfonyl)-N'-(4,6-dimethyl- pyrimidin-2-yl)-N"-methoxyguanidin nach Anspruch 1, gekennzeichnet durch die Formel

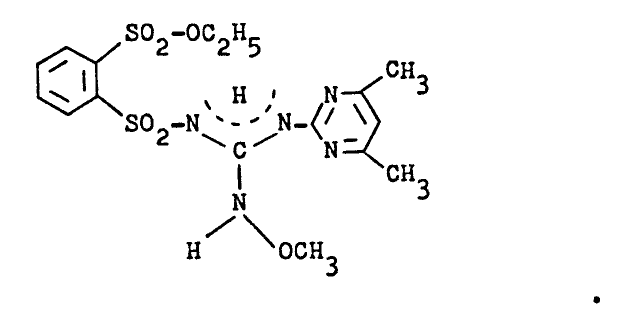

3. N-(2-Methoxysulfonylbenzolsulfonyl)-N'-(4,6-dimethyl- pyrimidin-2-yl)-N"-methoxyguanidin nach Anspruch 1, gekennzeichnet durch die Formel

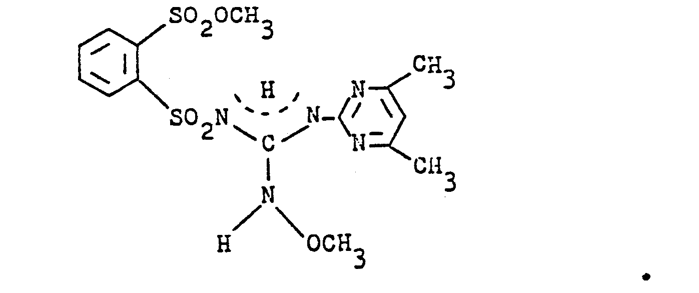

4. Verfahren zur Herstellung eines Sulfonylguanidin-Derivats der allgemeinen Formel 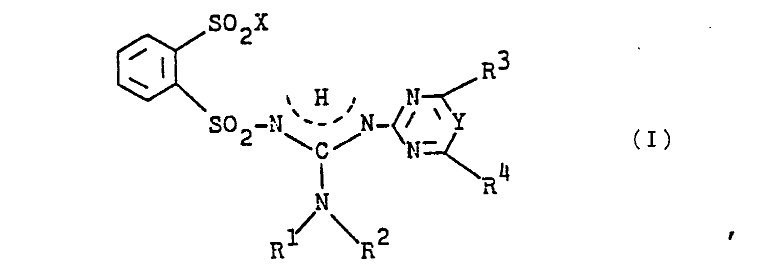 in der
X eine niedere Alkoxy-Gruppe, eine niedere Alkylamino-Gruppe oder eine Morpholino-Gruppe bezeichnet.
R¹ ein Wasserstoff-Atom oder die Gruppe 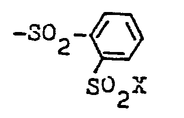 bezeichnet, in der X die oben angegebene Bedeutung hat,
_{R}² eine Hydroxy-Gruppe, eine niedere Alkoxy-Gruppe, eine Di-Niederalkylamino-Gruppe bezeichnet,
_{R}³ und _{R}⁴ jeweils für eine niedere Alkyl-Gruppe oder eine niedere Alkoxy-Gruppe stehen und
Y N oder CH bezeichnet, dadurch gekennzeichnet, daß eine Verbindung der Formel 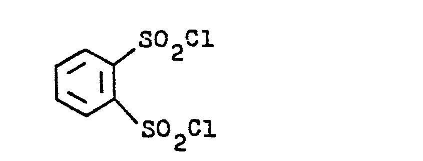 mit einer Verbindung der allgemeinen Formel 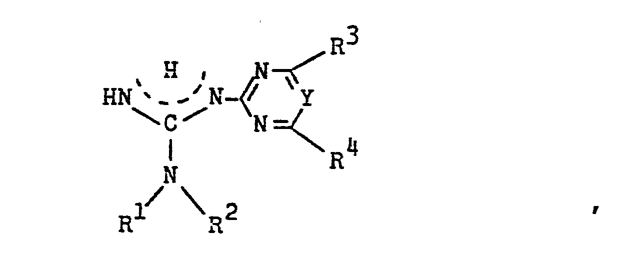 in der
_{R}¹, _{X}, _{R}², _{R}³, _{R}⁴ und Y die im Vorstehenden angegebenen Bedeutungen haben, in Gegenwart einer Base umgesetzt wird und anschließend das Produkt mit einer Verbindung der allgemeinen Formel 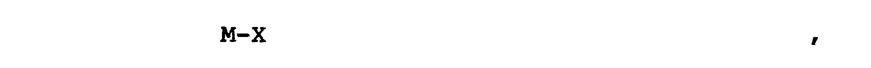 in der
X die im Vorstehenden angegebene Bedeutung hat und M ein Wasserstoff-Atom oder ein Alkalimetall-Atom bezeichnet, umgesetzt wird.

5. Verfahren zur Herstellung eines Sulfonylguanidin-Derivats der allgemeinen Formel 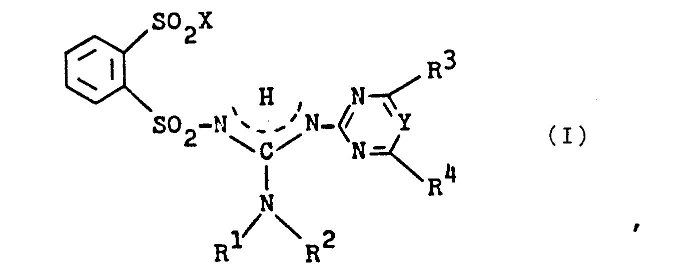 in der
X eine niedere Alkoxy-Gruppe, eine niedere Alkylamino-Gruppe oder eine Morpholino-Gruppe bezeichnet,
R¹ ein Wasserstoff-Atom oder die Gruppe 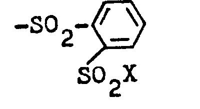 bezeichnet, in der X die oben angegebene Bedeutung hat,
R² eine _{H}ydroxy-Gruppe, eine niedere Alkoxy-Gruppe,
eine Di-Niederalkylamino-Gruppe bezeichnet, _{R}³ und _{R}⁴ jeweils für eine niedere Alkyl-Gruppe oder eine niedere Alkoxy-Gruppe stehen und
Y N oder CH bezeichnet, dadurch gekennzeichnet, daß eine Verbindung der allgemeinen Formel 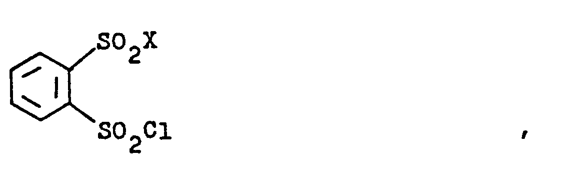 in der
X die im Vorstehenden angegebene Bedeutung hat, mit einer Verbindung der allgemeinen Formel 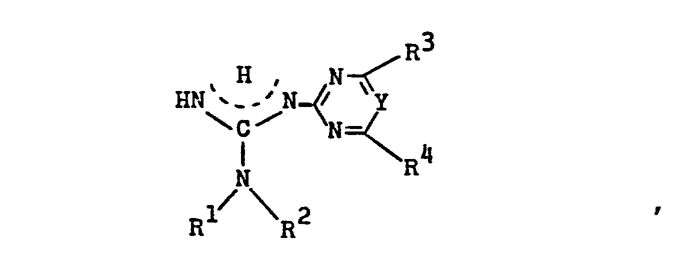 in der _{R}¹, _{X}, _{R}², _{R}³, _{R}⁴ und _{Y} die im Vorstehenden angegebenen Bedeutungen haben, in Gegenwart einer Base umgesetzt wird.

6. Verfahren zur Herstellung eines Sulfonylguanidin-Derivats der allgemeinen Formel 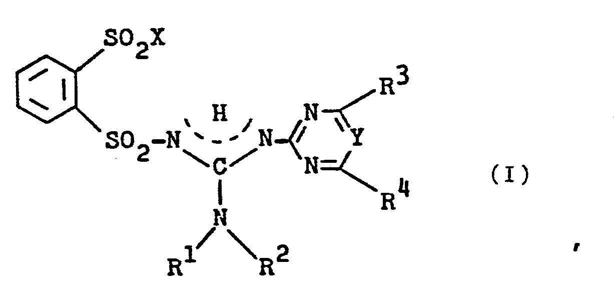 in der
X eine niedere Alkoxy-Gruppe, eine niedere Alkylamino-Gruppe oder eine Morpholino-Gruppe bezeichnet,
R¹ ein Wasserstoff-Atom oder die Gruppe 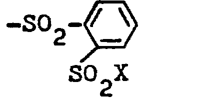 bezeichnet, in der X die oben angegebene Bedeutung hat,
R² eine Hydroxy-Gruppe, eine niedere Alkoxy-Gruppe, eine Di-Niederalkylamino-Gruppe bezeichnet,
_{R}³ und _{R}⁴ jeweils für eine niedere Alkyl-Gruppe oder eine niedere Alkoxy-Gruppe stehen und
Y N oder CH bezeichnet, dadurch gekennzeichnet, daß eine Verbindung der allgemeinen Formel 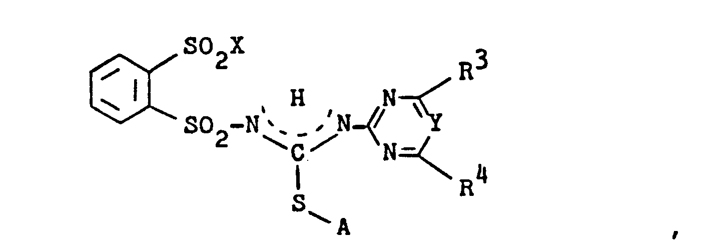 in der
_{X}, _{R}³, _{R}⁴, Y und M die im Vorstehenden angegebenen Bedeutungen haben und A eine niedere Alkyl-Gruppe bezeichnet,
mit einer Verbindung der allgemeinen Formel 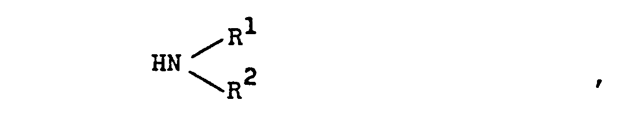
in der
_{R}¹ und R² die im Vorstehenden angegebenen Bedeutungen haben, umgesetzt wird.

7. Herbizide Mittel, gekennzeichnet durch einen Gehalt an mindestens einem Sulfonylguanidin-Derivat der allgemeinen Formel (I) gemäß Anspruch 1.

8. Verwendung von Sulfonylguanidin-Derivaten der allgemeinen Formel (I) gemäß Anspruch 1 zur Bekämpfung von unerwünschtem Pflanzenwachstum.

9. Verfahren zur Herstellung von herbiziden Mitteln, dadurch gekennzeichnet, daß man Sulfonylguanidin-Derivate der allgemeinen Formel (I) gemäß Anspruch 1 mit Streckmitteln und/oder oberflächenaktiven Mitteln vermischt.
